# FASCICULE DE BREVET EUROPEEN

(11) **EP 1 361 831 B1**
(45) Date de publication et mention de la délivrance du brevet: **25.07.2007**
(21) Numéro de dépôt: 02710745.7
(22) Date de dépôt: 19.02.2002
(51) Int. Cl.: A61C 5/02, B23C 5/10, A61B 17/16

(54) **INSTRUMENT D'ALESAGE, EN PARTICULIER POUR L'ALESAGE DE CANAUX DENTAIRES**
BOHRWERKZEUG, INSBESONDERE ZUR BEARBEITUNG VON ZAHNWURZELKANÄLEN
DRILLING INSTRUMENT, IN PARTICULAR FOR DRILLING DENTAL ROOT CANALS

(30) Priorité: 20.02.2001 FR 0102452
(43) Date de publication de la demande: 19.11.2003
(73) Titulaire: Rouiller, Jean-Claude, 2300 La Chaux-de-Fonds (CH)
(72) Inventeur: ROUILLER, Jean-Claude, CH-2300 La Chaux-de-Fonds (CH); BREGUET, Olivier, CH-2400 Le Locle (CH)
(74) Mandataire: Nithardt, Roland
(86) Numéro de dépôt international: PCT/CH2002/000098
(87) Numéro de publication internationale: WO 2002/065938

(56) Documents cités:
- WO-A-00/59399
- US-A- 4 260 379
- US-A- 6 074 209

## Description

### Domaine technique

La présente invention concerne un instrument d'alésage, en particulier pour l'alésage de canaux dentaires dans le cadre d'un traitement de racines dentaires, cet instrument comportant une base, un tronçon de coupe et un tronçon de guidage, le tronçon de coupe étant délimité par une enveloppe de forme cylindrique ou conique, ce tronçon de coupe étant pourvu de zones de dégagement, disposées en retrait par rapport à l'enveloppe, alternant avec des zones d'alésage disposées sur l'enveloppe.

### Technique antérieure

Actuellement, la plupart des instruments d'alésage de canaux dentaires a une partie active dite tronçon de coupe ayant une enveloppe conique et comporte une ou plusieurs arêtes de coupes enroulées en hélice le long de cette partie active.

Cette taille en hélice est indispensable pour l'évacuation des débris de dents vers l'extérieur de la racine. En cours d'utilisation, la partie conique peut subir un phénomène de gainage lorsque l'instrument est introduit dans le canal et, lorsqu'il est entraîné en rotation, il peut arriver qu'il se visse dans le canal. Pour cette raison, il est recommandé d'utiliser un tel instrument uniquement selon un mouvement axial manuel avec éventuellement un léger mouvement de rotation alternativement dans un sens et dans le sens inverse, faute de quoi, l'instrument peut se bloquer dans la dent et se casser. La rupture de l'instrument peut entraîner des conséquences graves étant donné qu'un canal dentaire est étroit et que l'accès est en est difficile.

Il existe certains instruments pour lesquels la tendance au vissage a été partiellement éliminée. Ceci a en particulier été obtenu en émoussant fortement les angles de coupe. Ce type d'instrument peut être utilisé avec un moteur d'entraînement en rotation tournant à faible vitesse afin de ne pas déformer la trajectoire naturelle du canal tout en l'élargissant.

Le fait que les arêtes de coupe soient émoussées constitue un inconvénient pour plusieurs raisons. D'une part le travail de coupe nécessaire pour élargir le canal dentaire s'effectue difficilement. D'autre part, la friction entre les arêtes émoussées et les parois du canal engendre un couple de travail important, ce qui peut également entraîner la rupture de l'instrument. Afin d'éviter ce risque, il est nécessaire d'utiliser toute une gamme d'instruments ayant différentes conicités, ceux ayant la conicité la plus grande étant utilisés les premiers. Dans ce cas, l'effet de gainage est éliminé et les contraintes dues à la friction, engendrées sur l'instrument restent inférieures à la limite de rupture. Toutefois, le travail ne s'applique que sur une partie limitée du canal dentaire.

Avec la plupart des instruments à entraînement motorisé connus le gain de temps par rapport à la méthode manuelle n'est pas certain et les risques de rupture restent importants.

Divers instruments de ce type ont fait l'objet de brevets. Le brevet américain US 6,074,209 décrit précisément un de ces instruments dont les arêtes sont émoussées. Il comporte des zones de section rétrécie qui alternent avec des zones de section plus large. Les zones de section rétrécie sont obtenues par meulage de la pièce en des endroits prédéterminés d'une ébauche dont la section est initialement constante si l'instrument est cylindrique, ou régulièrement décroissante si l'instrument a une forme générale conique, ce qui engendre des passages entre les zones de section rétrécie et les zones de section plus large à arêtes transversales vives qui risquent d'accrocher les parois du canal et de bloquer l'instrument.

La demande internationale publiée sous le N° WO 00/59399 décrit un instrument de ce type réalisé en torsadant une pièce allongée de forme générale conique et dont la section est en forme de losange. C'est également le cas de l'instrument décrit dans le brevet américain US 4,260,379. Le losange ayant une grande diagonale et une petite diagonale, l'instrument réalisé en torsadant une pièce dont la section est un losange comporte des zones alternatives de section réduite et de section plus grande correspondant respectivement à la petite et à la grande diagonale du losange. Ces zones de section différente ne sont pas décalées sur la longueur de l'instrument, mais elles sont angulairement décalées de 90 degrés. On a constaté que cette géométrie ne permet pas d'éviter le phénomène de vissage de l'instrument et que les contraintes exercées sur cet instrument pouvaient entraîner sa rupture.

### Exposé de l'invention

La présente invention se propose de pallier les inconvénients des instruments d'alésage de l'art antérieur en offrant un instrument dans lequel le risque de vissage est éliminé tout en maintenant un couple relativement faible évitant ainsi une fatigue prématurée.

Ces buts sont atteints par un instrument tel que défini dans la revendication 1.

Selon une forme de réalisation préférée, l'axe du tronçon de coupe est décalé par rapport à l'axe de ladite enveloppe. Cet axe du tronçon de coupe est hélicoïdal et s'enroule en hélice autour de l'axe rectiligne de l'enveloppe, ceci afin de rendre les zones d'évacuation plus profondes et plus efficaces.

D'une manière particulièrement avantageuse, une zone intermédiaire située entre la partie centrale d'une zone de dégagement et la partie centrale d'une zone d'alésage adjacente est divisée en n segments, où n correspond au nombre des arêtes du tronçon de coupe, et le long de chacun de ces segments le nombre d'arêtes disposées sur l'enveloppe est incrémenté d'une unité dans le sens allant d'une zone de dégagement vers la zone d'alésage adjacente.

Dans le cas particulier où ledit tronçon de coupe a une section triangulaire, une zone intermédiaire, située entre la partie centrale d'une zone de dégagement et la partie centrale d'une zone d'alésage adjacente, est de préférence divisée en trois segments et, le long de chacun de ces segments, le nombre d'arêtes disposées sur l'enveloppe passe successivement de zéro à un, puis à deux et enfin à trois dans le sens allant d'une zone de dégagement vers la zone d'alésage adjacente.

Selon un mode de réalisation particulièrement avantageux, au moins une goujure, qui est la face délimitée par deux arêtes du tronçon de coupe, est surtaillée par rapport aux autres de façon à former lesdites zones de dégagement.

Dans le cas où le tronçon de coupe comporte quatre arêtes de coupe, ces arêtes définissent quatre goujures qui sont disposées sensiblement à angle droit.

D'une manière préférentielle ledit instrument est utilisé dans le domaine de l'odontologie et la dimension de la zone de dégagement est inférieure de 0,1 mm à celle de la zone d'alésage.

Lorsque la section droite du tronçon de coupe est triangulaire, sa forme est de préférence celle d'un triangle équilatéral.

### Description sommaire des dessins

La présente invention et ses avantages seront mieux compris en référence à la description suivante de différents modes de réalisation de l'invention et aux dessins annexés, dans lesquels :
- la figure 1 est une vue d'ensemble de l'instrument selon la présente invention;
- la figure 2 est une vue agrandie d'une partie centrale de l'instrument de la figure 1 et correspondant au tronçon de coupe;
- les figures 3A et 3D sont des vues en coupe respectivement selon les lignes A-A et B-B de la figure 2 illustrant la variation du profil du tronçon de coupe, les figures 3B et 3C étant des vues intermédiaires entre ces deux positions;
- la figure 4 est une vue en coupe illustrant un mode de réalisation particulière du tronçon de coupe, et
- la figure 5 représente une autre forme de réalisation de l'instrument selon l'invention.

### Manières de réaliser l'invention

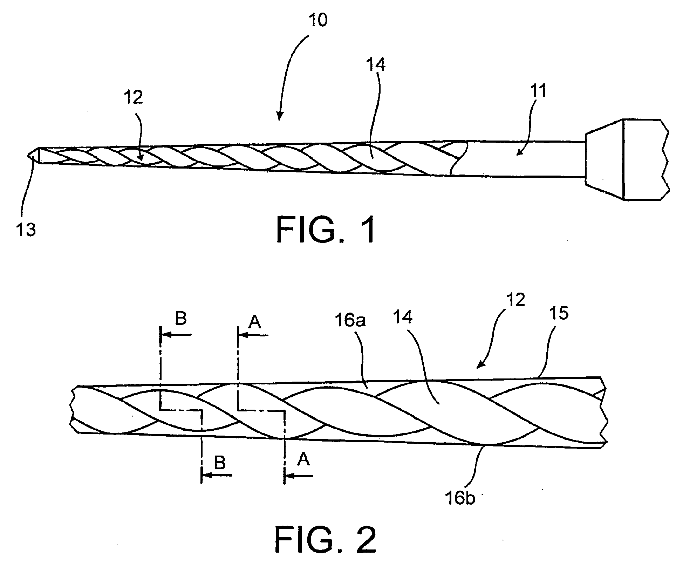

En référence aux figures l'instrument d'alésage 10 selon la présente invention comporte une base 11, un tronçon de coupe 12 et un tronçon de guidage 13. La base 11 est conventionnelle et peut avoir une forme cylindrique ou conique, avec une section circulaire ou polygonale et notamment carrée ou triangulaire. Le tronçon de guidage 13 est également formé de façon conventionnelle et se termine par une pointe arrondie permettant une introduction facile dans l'alésage à traiter et en particulier dans un canal dentaire. Dans l'exemple illustré par la figure 1, le tronçon de guidage 13 présente une certaine conicité qui permet à l'opérateur de suivre aisément le tracé naturel du canal en vue de son élargissement au moyen du tronçon de coupe 12.

Le tronçon de coupe 12 a une forme nouvelle par rapport aux tronçons de coupe des instruments existants. Dans les exemples illustrés, il est de section polygonale et comporte des arêtes vives. Plus particulièrement, dans l'exemple illustré par les figures 1 à 4, il comporte une section triangulaire formant des arêtes vives 17a, 17b et 17c définissant trois goujures 14 de forme hélicoïdale qui sont inscrites dans une enveloppe 15 sensiblement tronconique, une goujure étant la face délimitée par deux arêtes du tronçon de coupe 12. Ce tronçon de coupe 12 comporte des zones de dégagement 16a disposées en retrait par rapport à l'enveloppe 15 et qui alternent avec des zones d'alésage 16b disposées sur l'enveloppe, la dimension de la zone de dégagement 16a étant inférieure de 0,1 mm à celle de la zone d'alésage 16b.

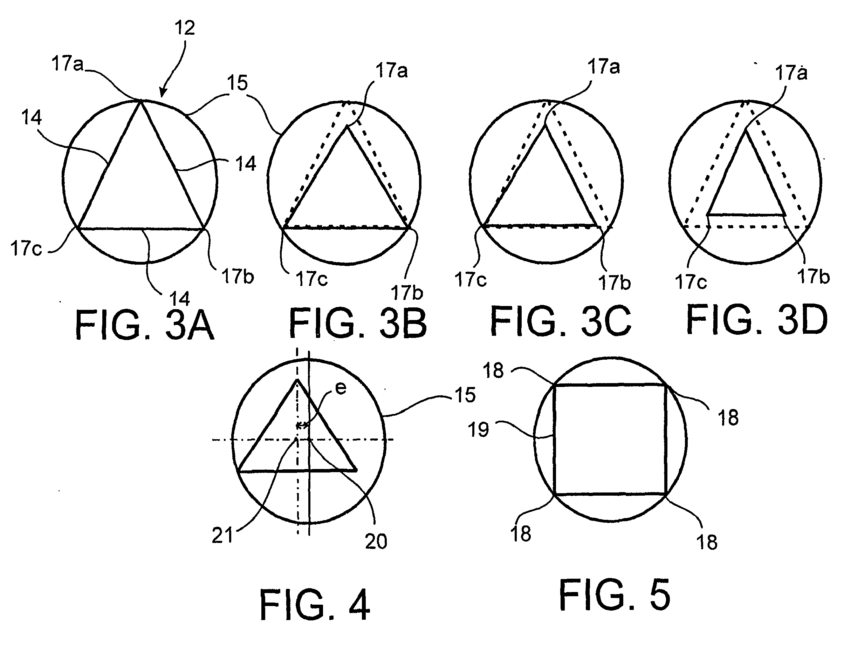

Comme le montre la figure 3A, qui est une vue en coupe dans la partie centrale d'une zone d'alésage, les arêtes 17a, 17b et 17c sont toutes disposées sur ladite enveloppe 15 et, comme le montre la figure 3D, qui est une vue en coupe dans la partie centrale d'une zone de dégagement, ces arêtes sont toutes disposées en retrait à l'intérieur de ladite enveloppe 15. Les figures 3B et 3C montrent que dans une zone intermédiaire entre une zone de dégagement et une zone d'alésage adjacente, au moins une arête 17b et/ou 17c est sur l'enveloppe et au moins une arête 17a et/ou 17b est en retrait à l'intérieur de l'enveloppe 15.

Puisque la section droite du tronçon de coupe 12 a une forme polygonale, le nombre de ses arêtes étant n, et en considérant qu'une zone intermédiaire située entre la partie centrale d'une zone de dégagement 16a et la partie centrale d'une zone d'alésage 16b adjacente est divisée en n segments, lorsqu'on se déplace le long de chacun de ces segments, le nombre d'arêtes disposées sur l'enveloppe 15 est incrémenté d'une unité dans le sens allant d'une zone de dégagement vers la zone d'alésage adjacente. De cette manière, le passage des arêtes de leur position sur l'enveloppe 15 vers une position en retrait par rapport à cette enveloppe s'effectue de façon continue et régulière.

Lorsque ledit tronçon de coupe 12 a une section triangulaire, une zone intermédiaire située entre la partie centrale d'une zone de dégagement 16a et la partie centrale d'une zone d'alésage 16b adjacente est divisée en trois segments et, le long de chacun de ces segments, le nombre d'arêtes disposées sur l'enveloppe passe successivement de zéro à un, puis à deux et enfin à trois dans le sens allant d'une zone de dégagement 16a vers la zone d'alésage 16b adjacente.

Pour réaliser cette géométrie faite au moyen d'un équipement de meulage, au moins une goujure 14 est surtaillée localement par rapport aux autres de façon à former lesdites zones de dégagement 16a. Au moins une partie de l'une des goujures est taillée de façon telle qu'elle n'est pas tangente à l'enveloppe.

Une variante illustrée par la figure 4 permet d'accroître encore l'efficacité des zones de dégagement. Selon cette réalisation, l'axe 20 de l'enveloppe 15 est décalé d'une distance e de l'axe 21 du tronçon de coupe 12. L'axe 20 de l'enveloppe est rectiligne et l'axe 21 du tronçon de coupe est hélicoïdal et s'enroule en hélice autour de l'axe 20. Cette réalisation permet d'approfondir les zones de dégagement et de les rendre plus efficaces pour l'entraînement de la matière en cours de traitement.

Dans l'exemple illustré par la figure 5, le tronçon de coupe 12 a une section rectangulaire et comporte donc quatre arêtes vives 18 qui définissent quatre goujures 19 disposées sensiblement à angle droit. Dans cette forme de réalisation deux goujures opposées peuvent être surtaillées.

Ces modes de réalisation présentent de nombreux avantages par rapport aux instruments similaires de l'art antérieur. D'une part, l'instrument est facile à usiner puisque l'existence d'une zone de dégagement espacée entre l'enveloppe et une ou plusieurs goujures ne modifie pratiquement pas le procédé de fabrication qui est basé sur le déplacement relatif des meules et de l'ébauche de l'instrument d'une manière automatique et programmée.

En outre, ils évitent de façon absolue tout risque de vissage de l'instrument. Ainsi, il est possible et même souhaitable de conserver une arête de coupe tranchante. Ceci permet un excellent usinage de la matière tout en évitant un échauffement de l'instrument et en maintenant un couple relativement faible. La durée de vie de l'instrument se trouve ainsi augmentée et le risque de rupture est fortement diminué.

La diminution du couple permet également d'utiliser un instrument relativement souple, ce qui assure son bon guidage dans un canal dentaire courbe.

Comme le risque de vissage est nul, il n'est pas nécessaire d'utiliser une grande gamme d'instruments ayant des conicités différentes, ce qui diminue, d'une part, le coût du matériel nécessaire à un traitement et, d'autre part, la durée de ce traitement.

### Possibilités d'application industrielle

La présente invention a été décrite essentiellement dans une application en odontologie, avec un foret de forme conique ou cylindrique. D'autres formes de forets pourraient également être utilisées. En particulier, il est possible d'appliquer l'invention à des forets de types connus sous les dénominations de foret « Gate », « Peeso » ou de forets cylindriques, ainsi que pour des fraises dites pour pivots, à os surtaillé ou « fissure ».

Le pas d'hélice des goujures, leur nombre et la dimension des zones de dégagement ne sont pas limités et il est possible de les modifier sans sortir du cadre de la présente invention.

Il est également possible d'utiliser l'instrument objet de l'invention dans d'autres domaines que l'odontologie, notamment en chirurgie et en particulier en orthopédie, ainsi que dans des domaines tels que la mécanique pour le travail de métaux ou de matières synthétiques, la menuiserie ou le travail du bois et des matériaux apparentés. Ceci permet notamment de réaliser des forets à usages manuels dans lesquels il n'y a pas de risque de vissage.

## Revendications

1. Instrument d'alésage, en particulier pour l'alésage de canaux dentaires, dans le cadre d'un traitement de racines dentaires, ledit instrument (10) comportant une base (11), un tronçon de coupe (12) et un tronçon de guidage (13), le tronçon de coupe étant délimité par une enveloppe (15) de forme cylindrique ou conique, ce tronçon de coupe étant pourvu de zones de dégagement (16a) disposées en retrait par rapport à l'enveloppe (15), alternant avec des zones d'alésage (16b) disposées sur ladite enveloppe, le tronçon de coupe (12) comporte des arêtes vives qui, dans la partie centrale d'une zone de dégagement (16a), sont toutes disposées en retrait à l'intérieur de l'enveloppe (15), et qui, dans la partie centrale d'une zone d'alésage (16b), sont toutes disposées sur ladite enveloppe, **caractérisé en ce que** le tronçon de coupe (12) est de section polygonale et **en ce que**, dans une zone intermédiaire entre une zone de dégagement (16a) et une zone d'alésage (16b) adjacente, au moins une arête est sur l'enveloppe et au moins une arête est en retrait à l'intérieur de l'enveloppe.

2. Instrument d'alésage selon la revendication 1, **caractérisé en ce que** l'axe (21) du tronçon de coupe (12) est décalé par rapport à l'axe (20) de ladite enveloppe (15).

3. Instrument d'alésage selon la revendication 2, **caractérisé en ce que** l'axe (21) du tronçon de coupe (12) est hélicoïdal et tourne autour de l'axe rectiligne (20) de l'enveloppe (15).

4. Instrument d'alésage selon la revendication 1, **caractérisé en ce qu'**une zone intermédiaire située entre la partie centrale d'une zone de dégagement (16a) et la partie centrale d'une zone d'alésage (16b) adjacente est divisée en n segments, où n correspond au nombre des arêtes du tronçon de coupe (12), et **en ce que** le long de chacun de ces segments le nombre d'arêtes disposées sur l'enveloppe (15) est incrémenté d'une unité dans le sens allant d'une zone de dégagement (16a) vers la zone d'alésage (16b) adjacente.

5. Instrument d'alésage selon la revendication 4, dans lequel ledit tronçon de coupe (12) a une section triangulaire, **caractérisé en ce qu'**une zone intermédiaire située entre la partie centrale d'une zone de dégagement (16a) et la partie centrale d'une zone d'alésage (16b) adjacente est divisée en trois segments, et **en ce que** le long de chacun de ces segments le nombre d'arêtes disposées sur l'enveloppe passe successivement de zéro à un, puis à deux et enfin à trois dans le sens allant d'une zone de dégagement (16a) vers la zone d'alésage (16b) adjacente.

6. Instrument d'alésage selon la revendication 5, **caractérisé en ce qu'**au moins une goujure (14), qui est la face délimitée par deux arêtes du tronçon de coupe (12), est surtaillée par rapport aux autres de façon à former lesdites zones de dégagement (16a).

7. Instrument d'alésage selon la revendication 1, **caractérisé en ce que** le tronçon de coupe (12) comporte quatre arêtes de coupe (18) qui définissent quatre goujures (17) disposées sensiblement à angle droit.

8. Instrument d'alésage selon la revendication 1, utilisé dans le domaine de l'odontologie, **caractérisé en ce que** la dimension de la zone de dégagement (16a) est inférieure de 0,1 mm à celle de la zone d'alésage (16b).

9. instrument d'alésage selon la revendication 1, **caractérisé en ce que** le tronçon de coupe (12) a une section transversale ayant une forme de triangle équilatéral.

10. Instrument d'alésage selon la revendication 1, **caractérisé en ce que** le tronçon de coupe (12) a une section transversale de forme sensiblement carrée.

## Claims

1. Drilling instrument, in particular for drilling dental canals as part of a dental root treatment, said instrument (10) including a base (11), a cutting section (12) and a guiding section (13), the cutting section being defined by an envelope (15) of a cylindrical or conical shape, this cutting section being provided with clearance zones (16a), arranged so that they are set back relative to the envelope (15), alternating with drilling zones (16b) arranged on said envelope, the cutting section (12) including sharp edges that are all arranged so that they are set back from the envelope (15), in the central part of a clearance zone (16a), and that are all arranged on said envelope, in the central part of a drilling zone (16b), **characterized in that** the cutting section (12) is polygonal in cross section, and **in that**, in a zone intermediate between a clearance zone (16a) and an adjacent drilling zone (16b), at least one edge is on the envelope and at least one edge is set back from the envelope.

2. Drilling instrument according to Claim 1, **characterized in that** the axis (21) of the cutting section (12) is moved relative to the axis (20) of said envelope (15).

3. Drilling instrument according to Claim 2, **characterized in that** the axis (21) of the cutting section (12) is helicoidal and turns around the rectilinear axis (20) of the envelope (15).

4. Drilling instrument according to Claim 1, **characterized in that** an intermediate zone located between the central part of a clearance zone (16a) and the central part of an adjacent drilling zone (16b) is divided into n segments where n corresponds to the number of edges of the cutting section (12), and **in that** along each of these segments the number of edges placed on the envelope (15) is increased by a unit in the direction going from a clearance zone (16a) towards the adjacent drilling zone (16b).

5. Drilling instrument according to Claim 4, in which said cutting section (12) has a triangular cross section, **characterized in that** an intermediate zone located between the central part of a clearance zone (16a) and the central part of an adjacent drilling zone (16b), is divided into three segments, and **in that** along each of these segments, the number of edges placed on the envelope goes successively from zero to one, then to two and finally to three in the direction going from a clearance zone (16a) towards the adjacent drilling zone (16b).

6. Drilling instrument according to Claim 5, **characterized in that** at least one flute (14), which is the surface bounded by two edges of the cutting section (12), is overtrimmed relative to the others so as to form said clearance zones (16a).

7. Drilling instrument according to Claim 1, **characterized in that** the cutting section (12) includes four cutting edges (18), which define four flutes (17) arranged approximately at right angles.

8. Drilling instrument according to Claim 1, used in the field of odontology, **characterized in that** the dimension of the clearance zone (16a) is 0,1 mm less than that of the drilling zone (16b).

9. Drilling instrument according to Claim 1, **characterized in that** the cutting section (12) has a cross section with the shape of an equilateral triangle.

10. Drilling instrument according to Claim 1, **characterized in that** the cutting section (12) has a cross section that is approximately square in shape.

## Patentansprüche

1. Bohrwerkzeug, insbesondere zur Bearbeitung von Zahnwurzelkanälen im Rahmen einer Zahnwurzelbehandlung, wobei das Werkzeug (10) eine Basis (11), einen Schneidabschnitt (12) und einen Führungsabschnitt (13) umfasst, wobei der Schneidabschnitt durch eine Umhüllung (15) mit zylindrischer oder konischer Form begrenzt ist, wobei dieser Schneidabschnitt mit Aussparungsbereichen (16a) versehen ist, die in Bezug auf die Umhüllung (15) zurückspringend angeordnet sind und die mit Bohrbereichen (16b) abwechseln, die an der Umhüllung angeordnet sind, wobei der Schneidabschnitt (12) scharfe Kanten umfasst, die im mittleren Teil eines Aussparungsbereichs (16a) jeweils zurückspringend innerhalb der Umhüllung (15) angeordnet sind und die im mittleren Teil eines Bohrbereichs (16b) jeweils an der Umhüllung angeordnet sind, **dadurch gekennzeichnet, dass** der Schneidabschnitt (12) einen polygonalen Querschnitt aufweist und dass in einem Zwischenbereich zwischen einem Aussparungsbereich (16a) und einem angrenzenden Bohrbereich (16b) mindestens eine Kante an der Umhüllung angeordnet ist und mindestens eine Kante zurückspringend innerhalb der Umhüllung angeordnet ist.

2. Bohrwerkzeug nach Anspruch 1, **dadurch gekennzeichnet, dass** die Achse (21) des Schneidabschnitts (12) in Bezug auf die Achse (20) der Umhüllung (15) verschoben ist.

3. Bohrwerkzeug nach Anspruch 2, **dadurch gekennzeichnet, dass** die Achse (21) des Schneidabschnitts (12) schraubenförmig ist und um die geradlinige Achse (20) der Umhüllung (15) gedreht ist.

4. Bohrwerkzeug nach Anspruch 1, **dadurch gekennzeichnet, dass** ein Zwischenbereich, der zwischen dem mittleren Teil eines Aussparungsbereichs (16a) und dem mittleren Bereich eines angrenzenden Bohrbereichs (16b) angeordnet ist, in n Segmente unterteilt ist, wobei n der Anzahl der Kanten des Schneidabschnitts (12) entspricht, und dass entlang von jedem dieser Segmente die Anzahl der Kanten, die an der Umhüllung (15) angeordnet sind, in der Richtung ausgehend von einem Aussparungsbereich (16a) hin zum angrenzenden Bohrbereich (16b) um eine Einheit erhöht wird.

5. Bohrwerkzeug nach Anspruch 4, wobei der Schneidabschnitt (12) einen dreieckigen Querschnitt aufweist, **dadurch gekennzeichnet, dass** ein Zwischenbereich, der zwischen dem mittleren Teil eines Aussparungsbereichs (16a) und dem mittleren Bereich eines angrenzenden Bohrbereichs (16b) angeordnet ist, in drei Segmente unterteilt ist und dass entlang von jedem dieser Segmente die Anzahl der Kanten, die an der Umhüllung (15) angeordnet sind, in der Richtung ausgehend von einem Aussparungsbereich (16a) hin zum angrenzenden Bohrbereich (16b) nacheinander von null auf eins, sodann auf zwei und schließlich auf drei erhöht wird.

6. Bohrwerkzeug nach Anspruch 5, **dadurch gekennzeichnet, dass** mindestens eine Nut (14), die die Fläche ist, die durch zwei Kanten des Schneidabschnitts (12) begrenzt wird, im Verhältnis zu den anderen größer geschnitten ist, um die Aussparungsbereiche (16a) zu bilden.

7. Bohrwerkzeug nach Anspruch 1, **dadurch gekennzeichnet, dass** der Schneidabschnitt (12) vier Schneidkanten (18) umfasst, die vier Nuten (17) definieren, die im Wesentlichen im rechten Winkel angeordnet sind.

8. Bohrwerkzeug nach Anspruch 1, das auf dem Gebiet der Zahnmedizin verwendet wird, **dadurch gekennzeichnet, dass** die Abmessung des Aussparungsbereichs (16a) um 0,1 mm kleiner ist als jene des Bohrbereichs (16b).

9. Bohrwerkzeug nach Anspruch 1, **dadurch gekennzeichnet, dass** der Schneidabschnitt (12) einen Querschnitt aufweist, der die Form eines gleichseitigen Dreiecks besitzt.

10. Bohrwerkzeug nach Anspruch 1, **dadurch gekennzeichnet, dass** der Schneidabschnitt (12) einen Querschnitt von im Wesentlichen quadratischer Form aufweist.
